# EUROPEAN PATENT APPLICATION

(11) **EP 2 644 689 A1**
(43) Date of publication of application: **02.10.2013**
(21) Application number: 11843415.8
(22) Date of filing: 24.11.2011
(51) Int. Cl.: C12M 1/00, A61K 35/14, C12M 3/00, C12N 1/02, C12N 5/078

(54) **METHOD AND MATERIAL FOR SEPARATING LEUKOCYTES OR MONONUCLEAR CELLS**

(30) Priority: 25.11.2010 JP 2010262558
(71) Applicant: Kaneka Corporation, Osaka 530-8288 (JP)
(72) Inventor: SATO Nobuhiko, Settsu-shi Osaka 566-0072 (JP); TSUKAMOTO Ayako, Settsu-shi Osaka 566-0072 (JP)
(74) Representative: TBK
(86) International application number: PCT/JP2011/077067
(87) International publication number: WO 2012/070622

(57) **Abstract**

An object of the present invention is to provide a separation system and a separation material for efficiently separating white blood cells or mononuclear cells from a biological fluid containing blood cell components. White blood cells or mononuclear cells can be efficiently separated by contacting a biological fluid containing blood cell components with a separation material having an average fiber diameter of at least 2.0 µm but not more than 6.0 µm and an air permeability coefficient M of at least 6.2 but not more than 35 to capture white blood cells on the separation material, and recovering the captured white blood cells or mononuclear cells using a detachment solution.

## Description

### TECHNICAL FIELD

The present invention relates to a separation material and separation methods for selectively recovering white blood cells or mononuclear cells from a biological fluid containing blood cell components.

### BACKGROUND ART

Recent rapid developments in hematology and scientific technology have contributed to the wide spread of treatment styles that involve separating only a blood fraction necessary for the treatment from a biological fluid such as whole blood, bone marrow, umbilical cord blood, or a tissue extract, and then administering it to a patient to produce improved therapeutic effects, and that do not involve administering fractions unnecessary for the treatment, thereby reducing side effects.

For example, one of the treatment styles is directed to blood transfusion. Red blood cell products are blood products used to treat hemorrhage, the lack of red blood cells, and the lack of oxygen caused by hypofunction of red blood cells. For red blood cell products, white blood cells, which may induce any side effect such as an abnormal immunoreaction or graft versus host disease (GVHD), are unnecessary, and thus they should be removed using a filter. In some cases, not only white blood cells but also platelets are removed.

On the other hand, platelet products are blood products used to treat patients with hemorrhage or hemorrhagic tendencies due to the lack of a blood coagulation factor. In order to prepare platelet products, unnecessary cells and components other than platelets are removed by centrifugation, and only desired platelet components are collected.

Also, hematopoietic stem cell transplants have recently become popular as treatment for leukemia or solid cancers. In the transplants, a white blood cell group including hematopoietic stem cells required for the treatment is separated and administered. As a source of the hematopoietic stem cells, umbilical cord blood has attracted attention in addition to bone marrow and peripheral blood because of its advantages such as small burden on donors and high proliferative ability of cells. Additionally, recent studies have suggested that menstrual blood is also rich in stem cells, and thus raised the possibility of being able to use menstrual blood, which has hitherto gone to waste, as a valuable source of stem cells.

As for bone marrow and peripheral blood, white blood cells should be separated and purified by removing unnecessary cells and then administered. As for umbilical cord blood, on the other hand, since umbilical cord blood banking for blood relatives, which requires the blood to be cryopreserved until use, has become popular, white blood cells are likewise separated and purified in order to prevent red blood cell hemolysis that may occur during cryopreservation.

As the method for separating white blood cells, there have been proposed a centrifugation method using a density gradient solution containing ficoll, and a centrifugation method using hydroxyethyl starch as a red blood cell sedimenting agent. However, for example, these methods have the problem of contamination of bacteria and other foreign matter because these methods cannot be performed in a closed system, and they further have the problem of taking a long time to perform.

Recently proposed cell separation methods that need not centrifugation use filter materials that capture only white blood cells without capturing red blood cells and platelets, to recover white blood cells (Patent Literatures 1 and 2). However, it is known that filters for capturing white blood cells are required to have a fiber diameter of less than 3 µm (Non Patent Literature 1). Actually, the separation materials used in the previous literatures have a fiber diameter of less than 2.5 µm (Patent Literatures 1, 2 and 3). This is because the conventional white blood cell removing filters are intended to remove as many white blood cells as possible. Unfortunately, although their white blood cell capturing ability is high, for example, these filters tend to become clogged in the process of treating a biological fluid, and also cause a pressure elevation in the process of recovering cells, thereby leading to a reduction in the cell recovery yield and technical problems.

Thus, the conventional white blood cell removing filters cannot work well as alternatives to white blood cell recovery filters.

### CITATION LIST

### PATENT LITERATURE

Patent Literature 1: JP-T 2001-518792
Patent Literature 2: WO 98/32840
Patent Literature 3: JP-A H10-313855

### NON PATENT LITERATURE

Non Patent Literature 1: Journal of the Society of Fiber Science and Technology, Japan, Vol. 61, No. 8, pp. 215-216 (2005)

### SUMMARY OF INVENTION

### TECHNICAL PROBLEM

An object of the present invention is to provide a highly efficient separation material for recovering white blood cells or mononuclear cells from a biological fluid containing blood cell components with high efficiency, without causing a pressure elevation, and to provide cell separation methods using the separation material.

### SOLUTION TO PROBLEM

The present inventors intensively studied for separation materials and cell separation methods that are able to efficiently separate white blood cells or mononuclear cells from a biological fluid, and are less likely to cause a pressure elevation, and consequently found that the use of a specific separation material enables white blood cells and mononuclear cells to be efficiently separated. Thus, the present invention was completed.

Specifically, the present invention relates to a separation material for separating white blood cells or mononuclear cells from a biological fluid, the separation material including a nonwoven fabric having an average fiber diameter of at least 2.0 µm but not more than 6.0 µm, and an air permeability coefficient M of at least 6.2 but not more than 35. Preferably, the nonwoven fabric is made of at least one selected from the group consisting of polyolefins, polyamides and polyesters. Preferably, the biological fluid is at least one selected from the group consisting of peripheral blood, umbilical cord blood, bone marrow, menstrual blood, and tissue extracts.

The present invention also relates to a cell separation container that includes a container provided with an inlet and an outlet for a biological fluid, wherein the above-described separation material is packed in the container. In the cell separation container, the separation material is preferably packed in the form of a single layer or a laminate of layers oriented in a direction of flow of the biological fluid, and it is also preferably packed in a state of being compressed in a direction of flow of the biological fluid. Preferably, the cell separation container is in the form of a column.

The present invention further relates to a cell separation method, including a step of contacting a biological fluid with the above-described separation material to separate white blood cells or mononuclear cells.

The present invention further relates to a method for separating white blood cells or mononuclear cells, which includes: a first step of contacting a biological fluid with the above-described cell separation container to capture white blood cells or mononuclear cells on the separation material; and a second step of recovering the white blood cells or mononuclear cells from the separation material using a detachment solution. In this cell separation method, preferably, the first step includes introducing the biological fluid through the inlet of the cell separation container and discharging the biological fluid through the outlet, and the second step includes introducing the detachment solution through the outlet of the cell separation container and recovering the white blood cells or mononuclear cells through the inlet. Preferably, the method further includes, after the first step and before the second step, a step of introducing physiological saline or a buffer through the inlet to remove contaminant components in the cell separation container. Preferably, the method further includes, before the first step, a step of contacting physiological saline or a buffer with the separation material. Preferably, the method further includes, before the first step, a step of fixing the inlet for a biological fluid of the cell separation container below the outlet for a biological fluid of the cell separation container. Preferably, the separation material substantially captures white blood cells and platelets, and substantially does not capture red blood cells. Preferably, the separated white blood cells or mononuclear cells include hematopoietic stem cells and/or mesenchymal stem cells.

Furthermore, the present invention relates to a cryopreservation method that includes placing cells obtained by any of the cell separation methods in a liquid nitrogen environment. Preferably, the liquid nitrogen environment is at -196°C to -30°C. In the cryopreservation method, at least one cryoprotective agent selected from the group consisting of dimethyl sulfoxide, dextran, albumin, and hydroxyethyl starch is preferably used. Preferably, 80% or more of cryopreserved stem cells are viable.

Moreover, the present invention relates to white blood cells, mononuclear cells or stem cells, obtained by any of the cell separation methods. Preferably, the stem cells include cells selected from the group consisting of: CD34+ cells; CD133+ cells; CD34- and CD133+ cells; CD34+ and CD133+ cells; CD34+ and CD133- cells; CD45-, CD44+, CD73+, and CD90+ cells; CD45-, CD235a-, CD33-, and CD7- cells; CD45+, CD133+, and CD117+ cells; CD45+, CD133-, and CD117+ cells; CD45+, CD133+, and CD164+ cells; CD45+, CD133-, and CD164+ cells; and CD45- and CD309+ cells. Preferably, the white blood cells include: CD45+ and CD164+ cells; or CD45+ and CD117+ cells.

### ADVANTAGEOUS EFFECTS OF INVENTION

The present invention allows for easy, rapid, and efficient separation of white blood cells or mononuclear cells from a biological fluid such as whole blood, bone marrow, umbilical cord blood, menstrual blood, or a tissue extract, without easily causing clogging and a pressure elevation.

The separation material and the cell separation methods of the present invention have multiple advantages in that they are less likely to cause clogging compared to the conventional techniques, and can recover white blood cells or mononuclear cells at a high recovery yield. Additionally, these techniques can be used to separate white blood cells or mononuclear cells from peripheral blood, umbilical cord blood, bone marrow, menstrual blood, or a tissue extract basically without performing a pre-treatment (e.g. buffy coat), although these techniques can be used after such a pre-treatment.

A filter constituted by a container packed with the separation material of the present invention can be used in the treatment in an aseptic closed system. The filter allows the recovery of a white blood cell- or mononuclear cell-containing liquid that contains a cell group that is rich in hematopoietic stem cells and mesenchymal stem cells, and thus can be used as a filter for the preparation of therapeutic cells for regenerative medicine such as leukemia treatment, cardiac muscle regeneration, and blood vessel regeneration.

The separation material of the present invention provides white blood cells that include only a remarkably low level of contaminating red blood cells. Such white blood cells will hardly be affected by hemolysis or the like even if they are cryopreserved until use. Additionally, since these white blood cells are separated in an aseptic manner, they can be amplified without being subjected to any process, to prepare cells. Accordingly, the separation material of the present invention is very useful for filters for the preparation of cell sources for regenerative medicine as well as for the preparation of blood transfusion products. Thus, the separation material of the present invention makes it possible to prepare highly safe therapeutic cells that have few side effects.

### BRIEF DESCRIPTION OF DRAWINGS

Fig. 1 shows the results of the recovery yield of white blood cells from a fresh bovine peripheral blood sample;
Fig. 2 shows the results of the recovery yield of white blood cells from a fresh human peripheral blood sample;
Fig. 3 shows the results of the recovery yield of white blood cells from a fresh swine bone marrow sample;
Fig. 4 shows the results of the recovery yield of white blood cells from a fresh bovine peripheral blood sample;
Fig. 5 shows the results of the recovery yield of white blood cells from a fresh human peripheral blood sample;
Fig. 6 shows the results of the recovery yield of white blood cells from a fresh swine bone marrow sample;
Fig. 7 is a schematic view of a column;
Fig. 8 is a photograph of colonies of hematopoietic stem cells;
Fig. 9 shows an example of a blood cell component separation system; and
Fig. 10 shows an example of a blood cell component separation system (the inlet is positioned below the outlet.)

### DESCRIPTION OF EMBODIMENTS

The following description is offered to illustrate the present invention in detail, but is not intended to limit the present invention.

The present invention relates to a separation material for separating white blood cells or mononuclear cells from a biological fluid, which includes a nonwoven fabric having an average fiber diameter of at least 2.0 µm but not more than 6.0 µm, and an air permeability coefficient M of at least 6.2 but not more than 35.

The separation material is formed as a fibrous separation material in terms of the length of time of contact between the material and a biological fluid, and specifically includes a nonwoven fabric that can be easily prepared or available. Methods for producing such a nonwoven fabric are roughly classified into wet methods and dry methods, and specific examples thereof include, but are not limited to, resin bonding, thermobonding, spunlacing, needle punching, stitch bonding, spunbonding, melt blowing and other production methods. For a nonwoven fabric having a fiber diameter of 10 µm or less, melt blowing and spunlacing are preferred. The nonwoven fabric may be subjected to calendering or a plasma treatment.

The fibers for the nonwoven fabric may suitably be so-called split fibers which are formed by splitting a multicomponent fiber into a plurality of fibers because they have a complex structure of entangled fibers and enhance the blood cell separation efficiencies.

The separation material may be used without being put in any container or may be put in a container provided with an inlet and an outlet for a biological fluid. For practical reasons, the latter manner in which the separation material is put in a container is preferred.

The fibers of the nonwoven fabric preferably have an average fiber diameter of at least 2.0 µm but not more than 6.0 µm, more preferably at least 2.5 µm but not more than 5.7 µm, and still more preferably at least 3. 5 µm but not more than 5.0 µm. If the average fiber diameter is less than 2.0 µm, the probability of clogging is likely to be high and thus the recovery yield is likely to be reduced. If the average fiber diameter is more than 6. 0 µm, the separation material tends to have a lower ability to capture white blood cells.

The average fiber diameter refers to the width of fiber in the perpendicular direction to the fiber axis. The fiber diameter can be determined by photographing the separation material made of a nonwoven fabric using a scanning electron microscope, measuring the diameters of fibers based on a scale on the photograph, and averaging the measured diameters. Namely, the fiber diameter herein means the average fiber diameter determined as described above, and is specifically the average of 50 or more fibers, preferably the average of 100 or more fibers. It should be noted that when, for example, multiple fibers are overlapped, some fibers hinder measurement of the width of a target fiber, or some fibers which remarkably differ in diameter are present, the data of these fibers are not used to calculate the fiber diameter.

The separation material preferably has an air permeability coefficient M of at least 6.2 but not more than 35, more preferably at least 7.0 but not more than 14.2, and still more preferably at least 9.2 but not more than 10.0. If the air permeability coefficient is less than 6.2, the separation material is likely to capture cells at a high density, resulting in a lower recovery performance. If the air permeability coefficient is more than 35, fewer cells are likely to be captured on the separation material.

The air permeability coefficient M is defined as a product of the air permeability (cc/cm² ·sec) and the thickness (mm) of the separation material, and is a practical parameter that is not affected by the thickness of the separation material. Specifically, the air permeability is a parameter that depends on the pore size of the separation material, and among separation materials having the same air permeability, a thinner separation material has a smaller actual air permeability, which means that when compared for the same thickness, its air permeability is smaller. Accordingly, the product of the air permeability and the thickness can be used as a parameter practically representing the pore size of the separation material.

The air permeability can be easily determined in accordance with or based on the Frazier method specified in JIS L1096-1999. The thickness can also be measured by means of various devices such as a digital caliper. It should be noted that these measurement methods are non-limiting examples for the method for determine the air permeability.

When the separation material has an average fiber diameter of at least 2.0 µm but not more than 6.0 µm, and an air permeability coefficient M of at least 6.2 but not more than 35, the separation material is able to efficiently separate white blood cells or mononuclear cells.

The materials that can be used for the separation material preferably include polyolefins, polyamides and polyesters, based on considerations of sterilization resistance and safety of cells. Examples of polyolefins include polypropylene, polyethylene, high-density polyethylene, and low-density polyethylene. Examples of polyamides include nylon. Examples of polyesters include polyethylene terephthalate and polybutylene terephthalate. Other examples include synthetic polymers such as polyvinyl alcohol, polyvinylidene chloride, rayon, vinylon, acrylics (e.g. polymethyl methacrylate, polyhydroxyethyl methacrylate, polyacrylonitrile, polyacrylic acid, polyacrylate), nylon, polyimide, aramid (e.g. aromatic polyamide), polyamide, cuprammonium rayon, carbons, phenolic resin, polyester, pulp, hemp, polyurethane, polystyrene and polycarbonate; natural polymers such as agarose, cellulose, cellulose acetate, chitosan and chitin; inorganic materials such as glass; and metals. In particular, polyethylene terephthalate, polybutylene terephthalate, polypropylene, acrylics, nylon, polyurethane and glass are preferred. One of these materials may be used alone, or any of these may be combined, mixed or fused, if necessary. In addition, molecules having affinity for specific cells, such as proteins, peptides, amino acids and saccharides, may be fixed to these materials, if necessary.

The term "biological fluid" is intended to include whole blood, peripheral blood, bone marrow, umbilical cord blood, menstrual blood, and tissue extracts, and any combinations thereof, and can include fluids obtained by rough separation of the foregoing. Examples of the animal origin of the biological fluid include mammals such as humans, bovines, mice, rats, swine, monkeys, dogs and cats.

The biological fluid may be pre-treated with an anticoagulant. Examples of the anticoagulant include citrate anticoagulants (e.g. ACD (acid-citrate-dextrose) solution, CPD (citrate-phosphate-dextrose) solution, CPDA (citrate-phosphate-dextrose-adenine) solution), heparin, low-molecular-weight heparin, Futhan (nafamostat mesilate), and EDTA. The storage conditions of the biological fluid are not particularly limited, as long as the conditions do not affect the intended uses of the fractions.

Specific examples of white blood cells and mononuclear cells that can be separated by the separation material include lymphocyte cells, monocytes, CD3+ cells, CD14+ cells, CD19+ cells, hematopoietic stem cells, and mesenchymal stem cells.

The present invention further relates to a cell separation container that includes a container provided with an inlet and an outlet for a biological fluid, wherein the separation material is packed in the container.

The shape, size, and material of the container in which the separation material is packed are not particularly limited. The container may have any shape (e.g. spherical, container-shaped, cassette-shaped, bag-shaped, tubular, or columnar). Specific preferred examples include, but are not limited to, a translucent tubular container having a volume of about 0.1 mL to 400 mL and a diameter of about 0.1 cm to 15 cm; and a quadratic prism-shaped container having a thickness of about 0.1 cm to 5 cm and having rectangular or square faces with sides having a length of about 0.1 cm to 20 cm.

Examples of the container form include cross-flow type containers and column type containers. Either the cross-flow type or the column type may be used, and the type of container is not particularly limited. Column type containers are preferred because they allow a recovery solution to be uniformly introduced. Conventional cell separation containers for capturing nucleated cells are of the cross-flow type in consideration of the fact that they are able to efficiently recover cells; however, they restrict usable detachment solutions to highly viscous solutions. In contrast, the combination of the separation material of the present invention and a column type container can avoid a decrease in the cell recovery yield and exhibit high separation performance even when a detachment solution having low viscosity is used.

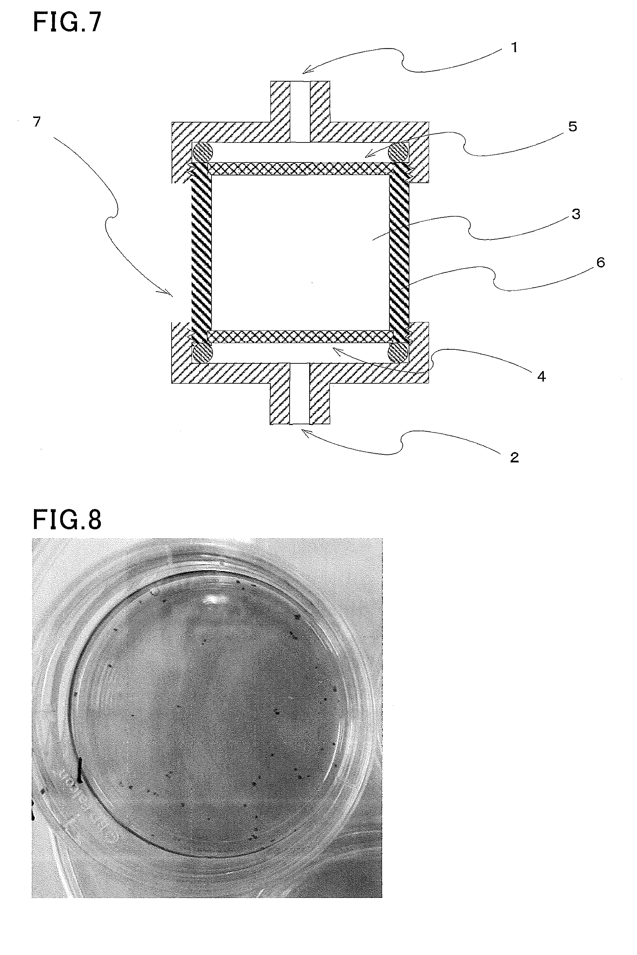

The column type refers to, for example, a container provided with an inlet and outlet for a liquid sample which are positioned around the center of the filter plane, a container provided with an inlet and outlet each oriented perpendicular to the filter plane, a container in which a liquid sample flows in the direction perpendicular to the filter plane, or a container in which a liquid sample flows in the parallel direction to the compression direction of the separation material. Fig. 7 shows one example of the column type.

On the other hand, the cross-flow type refers to a container whose inlet and outlet are positioned off the center of the filter plane, and each oriented parallel to the filter plane, as typified by the white blood cell removing filters ("SEPACELL" available from Asahi Kasei Medical Co., Ltd., "Purecell RC" available from Pall Corporation). The expression "inlet and outlet each oriented perpendicular to the filter plane" means that each of the inlet and outlet forms an angle (acute angle) of at least 45° but less than 90° with the filter plane, and the expression "inlet and outlet each oriented parallel to the filter plane" means that each of the inlet and outlet forms an angle (acute angle) of at least 0° but less than 45° with the filter plane.

The container may be made of any structural material. Specific examples of such structural materials include nonreactive polymers, biocompatible metals and alloys, and glasses. Examples of nonreactive polymers include acrylonitrile polymers (e.g. acrylonitrile butadiene styrene terpolymer), halogenated polymers (e.g. polytetrafluoroethylene, polychlorotrifluoroethylene, tetrafluoroethylene-hexafluoropropylene copolymer, polyvinyl chloride), polyamide, polyimide, polysulfone, polycarbonate, polyethylene, polypropylene, polyvinyl chloride-acrylic copolymer, polycarbonate acrylonitrile butadiene styrene, polystyrene, and polymethylpentene. Examples of usable metal materials (biocompatible metals and alloys) for the container include stainless steel, titanium, platinum, tantalum, gold, and alloys of these, gold plated ferroalloy, platinum plated ferroalloy, cobalt chromium alloy, and titanium nitride-coated stainless steel.

Materials having sterilization resistance are preferred among these, and specific examples thereof include polypropylene, polyvinyl chloride, polyethylene, polyimide, polycarbonate, polysulfone, and polymethylpentene.

Preferably, the separation material including a nonwoven fabric is cut into pieces of appropriate size, and they are used as a single layer or a laminate of layers, having a thickness of about 1 mm to 200 mm and oriented in a direction of flow of a biological fluid. Based on a consideration of the separation efficiencies of fractions, the thickness of such a laminate is more preferably 1.5 mm to 150 mm, and still more preferably 2 mm to 100 mm.

The separation material can be packed in the container, in the form of a single layer or a laminate of layers oriented in a direction of flow of a biological fluid, and the thickness in this state is preferably about 1 mm to 50 mm. Based on a consideration of the separation efficiencies of fractions, the thickness is more preferably 1.5 mm to 40 mm, and still more preferably 2 mm to 35 mm.

The separation material may be rolled up and then packed into the container. In the case that the separation material is used in the rolled-up form, a biological fluid may be treated while passing through this roll from the inside to the outside or conversely from the outside to the inside to separate blood cells.

When the separation material is packed into the container, the separation material may be packed in the container, in a state of being compressed in a direction of flow of a biological fluid, or may be packed in the container without being compressed. To be compressed or not can be appropriately selected according to the material of the separation material or other factors.

A flat sheet of appropriate size may be cut out from the separation material and then packed into the cell separation container, or the separation material may be rolled up and then packed into the container. Two or more kinds of separation materials may be used together, and the above-mentioned separation material may be used in combination with different separation material (s), as long as a separation system capable of substantially capturing and recovering white blood cells can be constructed. To substantially capture white blood cells means that the separation material captures 60% or more of white blood cells contained in a biological fluid when the separation material is contacted with the biological fluid. Moreover, it is preferable that the white blood cells captured on the separation material of the present invention constitute 60% or more of the white blood cells captured in the entire cell separation container.

The cell separation container is characterized in that the cell separation container substantially does not capture red blood cells, but substantially captures white blood cells. The expression "substantially does not capture red blood cells" means a characteristic such that when a biological fluid is contacted with the separation material, 60% or more of red blood cells in the biological fluid pass through the separation material. Moreover, the separation material of the present invention may be made of, but not limited to, a material capable of substantially capturing platelets. The expression "substantially capturing platelets" means that the separation material captures 50% or more of platelets contained in a biological fluid when the separation material is contacted with the biological fluid. More preferably, 60% or more of platelets are captured on the separation material.

The present invention also relates to a cell separation method that includes a step of contacting a biological fluid with the above-described separation material to separate white blood cells or mononuclear cells. The present invention further relates to a cell separation method that includes a first step of contacting a biological fluid with the above-described cell separation container to capture white blood cells or mononuclear cells on the separation material; and a second step of recovering the captured white blood cells or mononuclear cells from the separation material using a detachment solution.

Specifically, in the first step, a biological fluid is injected into the container packed with the separation material through the inlet of the container, and then white blood cells or mononuclear cells are captured while allowing red blood cells to pass through the separation material. In the subsequent second step, a detachment solution is passed through the container from the outlet side of the container, i.e., in the opposite direction to the direction of flow of the biological fluid and a washing solution, so that the white blood cells or mononuclear cells captured on the separation material can be separated and recovered at a high yield. After the step of contacting a biological fluid with the separation material to capture white blood cells on the separation material, a washing solution may be passed through the container in the same direction to efficiently separate and recover red blood cells remaining in the container, although it is not necessary to do so.

In the case where a biological fluid is passed through a cell separation container provided with an inlet and an outlet for a biological fluid, the inlet for a biological fluid may be set to be above the outlet for a biological fluid so that the biological fluid can flow in the direction of gravity. Alternatively, the inlet for a biological fluid may be set to be below the outlet for a biological fluid so that the biological fluid can flow in a direction opposite to gravity. In the case where the biological fluid is allowed to flow in a direction opposite to gravity, the biological fluid flows uniformly in the entire container, which further improves the separation efficiencies.

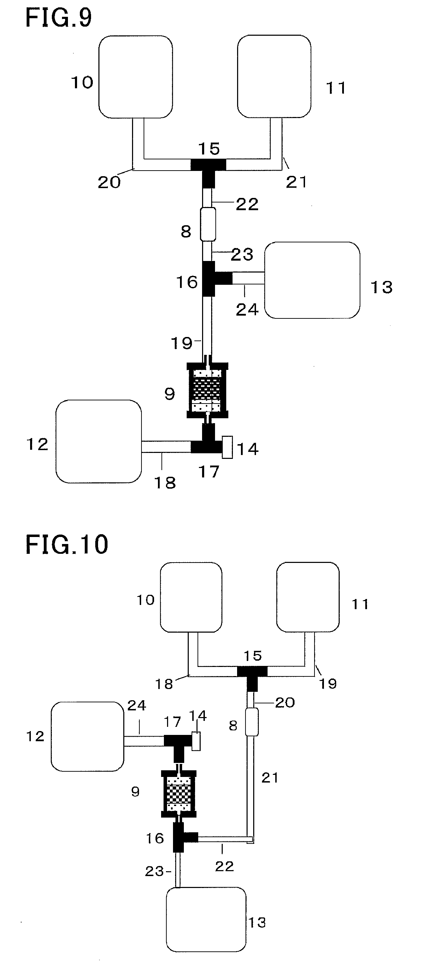

A blood cell separation component system can be constructed using the cell separation container. For practical reasons, the blood cell separation system preferably further includes, in addition to the cell separation container, inlets and outlets for a washing solution and a detachment solution, a red blood cell recovery bag, a white blood cell recovery bag, and the like. In this case, the cell separation container has an inlet through which a biological fluid enters the container, and an outlet through which the biological fluid is discharged, and preferably further includes: an inlet for a washing solution for washing out red blood cells remaining in the container, which is independent of the inlet for a biological fluid; an outlet for a washing solution, which is independent of the outlet for a biological fluid; and an inlet for introducing a detachment solution, which is independent of the inlets and outlets for a biological fluid and a washing solution. The inlet and outlet for a biological fluid of the container may also be used as the inlet and outlet for a washing solution, respectively, and a line on the inlet side may be connected to both a blood bag and a washing solution bag via a three-way stopcock or the like. The outlet for a biological fluid may also be used as the inlet for introducing a detachment solution. The inlet for a biological fluid may also be used as a detachment solution recovery side which may likewise be connected to bags, syringes, or the like via a three-way stopcock. Figs. 9 and 10 show examples of the blood cell separation system.

Preferably, a biological fluid storage bag, a detachment solution recovery bag for recovering separated white blood cells, a red blood cell recovery bag, and the like are also provided with the blood cell separation system. When these bags are connected to the inlet(s) and outlet(s) for the aforementioned solutions, a biological fluid can be subjected to separation in an aseptic closed system. Preferably, these bags after use can be cut off and then used. These bags may each have a shape like that of a commonly used blood bag or may each be in the form of a flat sheet cartridge or the like. The form of the bags may be selected from, for example, a bag usable for cell culture and a cryopreservation resistant bag, according to the purpose.

The following description is offered to specifically describe the separation methods of the present invention.

### 1) Biological fluid feeding process

In order to allow a biological fluid to pass through the container packed with the separation material from the biological fluid inlet, the biological fluid in another container may be fed from that container through a fluid feed line either by free fall under gravity or by a pump. Alternatively, a syringe containing the biological fluid may be directly connected to the container and then pressed by hand. In the case that the biological fluid is flowed by a pump, too high a feed rate tends to lead to low separation efficiencies, whereas too low a feed rate tends to elongate the treatment time. For example, the feed rate may be, but not limited to, 0.1 mL/min to 100 mL/min.

A process of soaking the separation material with physiological saline or a buffer may be performed as a pre-treatment prior to the biological fluid feeding process. This process is not essential but may be optionally performed because the soaking of the separation material with such a solution is expected to contribute to increasing the separation efficiencies and securing blood flow paths. The pre-treatment solution may not be the same as that used in the washing process described later, and it is preferably the same for simplicity of the line system and workability because the same solution bag can be shared. For practical reasons, the volume of the pre-treatment solution is preferably about 1 to 100 times the capacity of the container packed with the separation material. Any buffer can be used without particular limitations, and common buffers such as Ringer's solution, media for cell culture, and phosphate buffer are preferred.

### 2) Washing process

This process is not essential but may be performed to improve the efficiency of removing contaminants. Examples of contaminants that can be removed in this process include red blood cells and components other than blood cells such as plasma. In order to flow a washing solution from the washing solution inlet in the same direction as the biological fluid feeding direction, the washing solution may be fed through a line either by free fall under gravity or by a pump. In the case that the biological fluid is fed by a pump, the flow rate is similar to the flow rate in the biological fluid feeding process, and may be specifically, but not limited to, 0.1 mL/min to 100 mL/min. The volume of the washing solution depends on the capacity of the container. Too little washing solution may leave more red blood cell components in the container, whereas too much washing solution may lead to low separation efficiencies and remarkably elongate the process time. Based on these considerations, the volume of the washing solution is preferably set to about 0.5 to 100 times the capacity of the container.

Any washing solution can be used as long as it is able to wash out only red blood cells, reduce the contamination of the recovered white blood cells with other blood cells, and maintain the capture of the target blood cells. Preferred are common buffers such as physiological saline, Ringer' s solution, media for cell culture, and phosphate buffer.

### 3) Detachment of white blood cells or mononuclear cells

A detachment solution is injected into the container packed with the separation material in a direction opposite to the biological fluid flow direction (from the side from which the biological fluid is discharged), so that white blood cells are detached. The injection of the detachment solution can be accomplished by putting the detachment solution in a syringe or the like and then strongly pressing a plunger of the syringe by hand or by using an instrument. The volume and the flow rate of the recovery solution depend on the capacity of the container and the treatment amount. Preferably, the volume is about 1 to 100 times the capacity of the container and the flow rate is 0.5 mL/sec to 20 mL/sec although the volume and the flow rate are not limited to these ranges.

The detachment solution is not particularly limited, as long as it is a hypotonic solution. Examples thereof include solutions that have been used for injection (e.g. physiological saline, Ringer's solution, dextran injection, hydroxyethyl starch), buffers, and media for cell culture.

In order to enhance the recovery yield of captured cells, the viscosity of the recovery solution may be increased. For this purpose, to the detachment solution may be added a substance such as, but not limited to, albumin, fibrinogen, globulin, dextran, hydroxyethyl starch, hydroxyethyl cellulose, collagen, hyaluronic acid, and gelatin. The viscosity of the detachment solution is not particularly limited, and is preferably not more than 20 mPa·s because a highly viscous solution tends to make the recovery process difficult. In the case of a column type container, the viscosity may be 10 mPa·s or lower because the separation performance of such a container is not degraded even when a detachment solution having low viscosity is used. A detachment solution having a viscosity of 5 mPa·s or lower can also be used.

The white blood cells recovered by the cell separation methods preferably include hematopoietic stem cells, mesenchymal stem cells, and/or CD34+ cells.

The present invention further relates to a cryopreservation method that includes placing cells obtained by any of the cell separation methods in a liquid nitrogen environment. The cell separation methods applies very little stress to cells compared with conventional centrifugation methods, and the cells obtained by these cell separation methods maintain very high activity after cryopreservation.

Before cryopreservation of cells, a cryoprotective agent is added to protect cells under cryopreservation. The cryoprotective agent to be added is not particularly limited and examples include dimethyl sulfoxide, dextran, albumin, and hydroxyethyl starch. Any of these cryoprotective agents may be used alone, or two or more of these may be used in combination.

In the cryopreservation method, the cells have only to be stored in a liquid nitrogen environment, and specifically, may be immersed in liquid nitrogen and then stored, or may be stored in liquid nitrogen gas. The temperature during the storage is not particularly limited, and is preferably -196°C to -30°C in order to avoid a decrease in the activity of cells. The temperature is more preferably -196°C to -50°C, and still more preferably -196°C to -70°C.

The present invention further relates to white blood cells, mononuclear cells or stem cells, obtained by the cryopreservation method. The stem cells obtained by the cryopreservation method may be any stem cells as long as they are cells in a biological fluid and have self-renewal ability and differentiation potential. Specific examples include hematopoietic stem cells, mesenchymal stem cells, embryonic-like stem cells, and endothelial progenitor cells.

Examples of hematopoietic stem cells include CD34+ cells; CD133+ cells; CD34- and CD133+ cells; CD34+ and CD133+ cells; CD34+ and CD133- cells; CD45+, CD133+, and CD117+ cells; CD45+, CD133-, and CD117+ cells; CD45+, CD133+, and CD164+ cells; and CD45+, CD133-, and CD164+ cells.

Among hematopoietic stem cells, CD34+ cells and CD133+ cells are generally mentioned. Hematopoietic stem cells in umbilical cord blood are thought to differentiate into CD34- and CD133+ cells; CD34+ and CD133+ cells; and CD34+ and CD133-cells. The separation material of the present invention can be used to separate these hematopoietic stem cells, and the separated cells, when cryopreserved, will show only a slight decrease in the activity. Among these hematopoietic stem cells, in particular, CD117+ cells, which are receptors for a hemopoietic growth factor (stem cell factor) and are thought to be relevant to graft survival, and CD164+ cells, which are relevant to cell adhesion, will show only a slight decrease in the activity when they are separated by the separation material of the present invention and then cryopreserved.

Likewise, CD45-, CD44+, CD73+, and CD90+ cells, which are thought to be mesenchymal stem cells in umbilical cord blood, will show only a slight decrease in the activity when they are separated by the separation material of the present invention and then cryopreserved. Only a small amount of mesenchymal stem cells are present in umbilical cord blood, and thus there is the problem that these mesenchymal stem cells may be largely lost and cannot be easily separated by centrifugation; in contrast, the separation material of the present invention can efficiently separate these mesenchymal stem cells.

Interesting stem cells having multi-differentiation potential called lineage negative stem cells are present in umbilical cord blood. These cells are known as embryonic-like stem cells. The separation material of the present invention can efficiently separate these CD45-, CD235a-, CD33-, and CD7-cells, and the resulting cells will show only a slight decrease in the activity when they are cryopreserved.

Likewise, CD45- and CD309+ cells, which are endothelial progenitor cells, will show only a slight decrease in the activity when they are separated by the separation material of the present invention and then cryopreserved.

Additionally, CD45+ and CD164+ cells, and CD45+ and CD117+ cells, among white blood cells, will also show only a slight decrease in the activity when they are separated by the separation material of the present invention and then cryopreserved.

The viability of cells after cryopreservation is preferably not less than 80%, and more preferably not less than 85%.

### EXAMPLES

The following examples are offered to demonstrate the present invention in more detail. It should be noted that the present invention is not limited only to these examples. In the following Examples and Comparative Examples, the numbers of layers of each nonwoven fabric packed were adjusted such that all the uncompressed thicknesses were in a certain range. The containers packed with a separation material of Examples and Comparative Examples were each a column type container as shown in Fig. 7. Each container was provided with an inlet and an outlet for a liquid sample which were positioned at the center of the filter plane and were each oriented perpendicular to the filter plane.

### (Example 1)

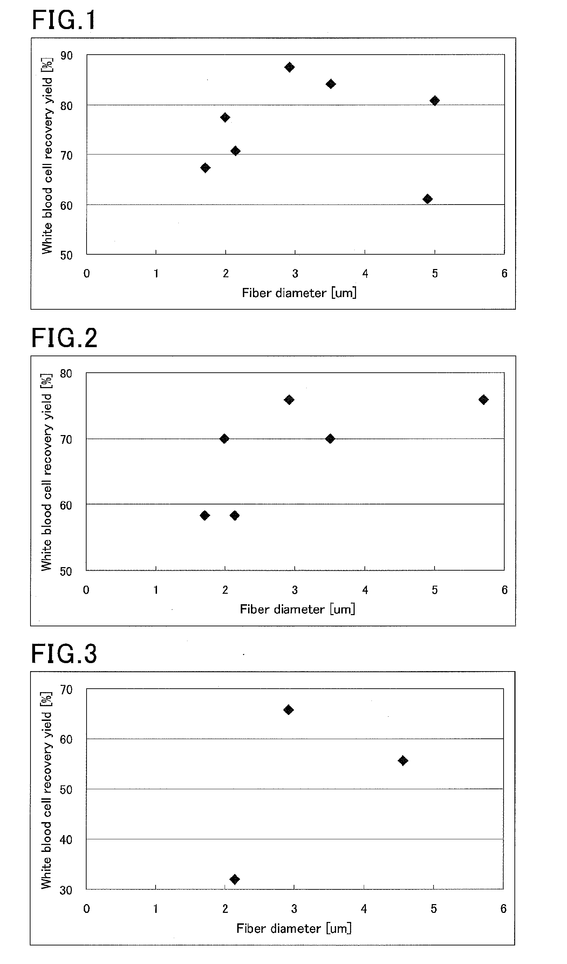

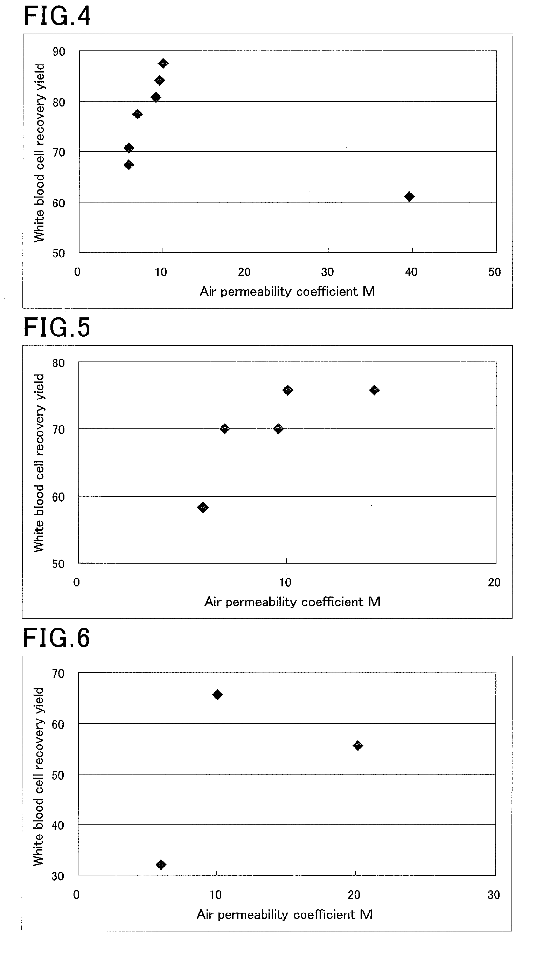

A laminate of 28 layers of a polybutylene terephthalate nonwoven fabric (average fiber diameter: 2.0 µm, air permeability coefficient M: 7.0) was packed into a polycarbonate column container (thickness: 6 mm, diameter: 18 mm), as shown in Fig. 7. First, physiological saline (45 mL) was passed through the container from the inlet side by pressing a syringe by hand. Next, CPD-anticoagulated fresh bovine blood (20 mL) (12% CPD-containing bovine peripheral blood (CPD:blood = 200:28)) was passed through the container at 2.5 mL/min, and then physiological saline (10 mL) was passed through the container in the same direction. Subsequently, αMEM supplemented with 10% FBS (30 mL) (viscosity: 2.9 mPa·s) was passed through the container in a direction opposite to the above flow direction by pressing a syringe by hand to recover white blood cells. The detachment solution could be smoothly fed into the container. The blood sample before the treatment and the recovered solution were evaluated for blood count using a blood cell counter (K-4500 available from Sysmex Corp.), and the white blood cell recovery yield was calculated. Table 1 and Figs. 1 and 4 show the results.

### (Example 2)

The same procedures as in Example 1 were carried out, except that a laminate of 28 layers of a polypropylene nonwoven fabric (average fiber diameter: 3.5 µm, air permeability coefficient M: 9.6) was packed. Table 1 and Figs. 1 and 4 show the results.

### (Example 3)

The same procedures as in Example 1 were carried out, except that a laminate of 28 layers of a polybutylene terephthalate nonwoven fabric (average fiber diameter: 2. 9 µm, air permeability coefficient M: 10.0) was packed. Table 1 and Figs. 1 and 4 show the results.

### (Example 4)

The same procedures as in Example 1 were carried out, except that a laminate of 32 layers of a nylon nonwoven fabric (average fiber diameter: 5.0 µm, air permeability coefficient M: 9.2) was packed. Table 1 and Figs. 1 and 4 show the results.

### (Comparative Example 1)

The same procedures as in Example 1 were carried out, except that a laminate of 84 layers of a polybutylene terephthalate nonwoven fabric (average fiber diameter: 1.7 µm, air permeability coefficient M: 5.9) was packed. Slight resistance was felt in the recovery process, which suggests that the inner pressure of the column was high and clogging was caused. Table 1 and Figs. 1 and 4 show the results.

### (Comparative Example 2)

The same procedures as in Example 1 were carried out, except that a laminate of 30 layers of a polypropylene nonwoven fabric (average fiber diameter: 2.1 µm, air permeability coefficient M: 6.0) was packed. Table 1 and Figs. 1 and 4 show the results.

### (Comparative Example 3)

The same procedures as in Example 1 were carried out, except that a laminate of 24 layers of a polypropylene nonwoven fabric (average fiber diameter: 4.9 µm, air permeability coefficient M: 39.6) was packed. Table 1 and Figs. 1 and 4 show the results.

**[Table 1]**

| | Nonwoven fabric material | Average fiber diameter [µm] | Air permeability coefficient M | Number of laminated layers | Biological fluid | White blood cell recovery yield [%] |
|---|---|---|---|---|---|---|
| Example 1 | Polybutylene terephthalate | 2.0 | 7.0 | 28 | Fresh bovine blood | 77 |
| Example 2 | Polypropylene | 3.5 | 9.6 | 28 | Fresh bovine blood | 84 |
| Example 3 | Polybutylene terephthalate | 2.9 | 10.0 | 28 | Fresh bovine blood | 88 |
| Example 4 | Nylon | 5.0 | 9.2 | 32 | Fresh bovine blood | 81 |
| Comparative Example 1 | Polybutylene terephthalate | 1.7 | 5.9 | 84 | Fresh bovine blood | 67 |
| Comparative Example 2 | Polypropylene | 2.1 | 6.0 | 30 | Fresh bovine blood | 71 |
| Comparative Example 3 | Polypropylene | 4.9 | 39.6 | 24 | Fresh bovine blood | 61 |

### (Example 5)

The same separation material as in Example 1 was used and the same procedures were carried out, except that CPD-anticoagulated fresh human blood (10 mL) was used instead of the CPD-anticoagulated fresh bovine blood (20 mL). Then, portions of the blood sample before the treatment and the recovered detachment solution were hemolyzed with FACS Lysing Solution and evaluated for mononuclear cell positivity using a flow cytometer (BD FACSCanto). The total number of mononuclear cells was calculated by multiplying the number of white blood cells by the mononuclear cell positivity. The mononuclear cell recovery yield was given as a percentage calculated by dividing the total number of mononuclear cells in the recovered solution by the total number of mononuclear cells before the treatment. Table 2 and Figs. 2 and 5 show the results.

### (Example 6)

The same separation material as in Example 2 was used and the same procedures were carried out, except that CPD-anticoagulated fresh human blood (10 mL) was used instead of the CPD-anticoagulated fresh bovine blood (20 mL). Table 2 and Figs. 2 and 5 show the results.

### (Example 7)

The same separation material as in Example 3 was used and the same procedures were carried out, except that CPD-anticoagulated fresh human blood (10 mL) was used instead of the CPD-anticoagulated fresh bovine blood (20 mL). Portions of the blood sample before the treatment and the recovered detachment solution were hemolyzed with FACS Lysing Solution and evaluated for mononuclear cell positivity using a flow cytometer (BD FACSCanto). The total number of mononuclear cells was calculated by multiplying the number of white blood cells by the mononuclear cell positivity. The mononuclear cell recovery yield was given as a percentage calculated by dividing the total number of mononuclear cells in the recovered solution by the total number of mononuclear cells before the treatment. Table 2 and Figs. 2 and 5 show the results.

### (Example 8)

The same procedures as in Example 7 were carried out, except that a laminate of 40 layers of a polypropylene nonwoven fabric (fiber diameter: 5.7 µm, air permeability coefficient M: 14.2) was packed instead of the polybutylene terephthalate nonwoven fabric (average fiber diameter: 2.9 µm, air permeability coefficient M: 10.0). Table 2 and Figs. 2 and 5 show the results.

### (Comparative Example 4)

The same separation material as in Comparative Example 1 was used and the same procedures were carried out, except that CPD-anticoagulated fresh human blood (10 mL) was used instead of the CPD-anticoagulated fresh bovine blood (20 mL). Slight resistance was felt in the recovery process, which suggests that the inner pressure of the column was high and clogging was caused. Table 2 and Figs. 2 and 5 show the results.

### (Comparative Example 5)

The same separation material as in Comparative Example 2 was used and the same procedures were carried out, except that CPD-anticoagulated fresh human blood (10 mL) was used instead of the CPD-anticoagulated fresh bovine blood (20 mL). Table 2 and Figs. 2 and 5 show the results.

**[Table 2]**

| | Nonwoven fabric material | Average fiber diameter [µm] | Air permeability coefficient M | Number of laminated layers | Biological fluid | White blood cell recovery yield [%] | Mononuclear cell recovery yield [%] |
|---|---|---|---|---|---|---|---|
| Example 5 | Polybutylene terephthalate | 2.0 | 7.0 | 28 | Fresh human blood | 70 | 90 |
| Example 6 | Polypropylene | 3.5 | 9.6 | 28 | Fresh human blood | 70 | - |
| Example 7 | Polybutylene terephthalate | 2.9 | 10.0 | 28 | Fresh human blood | 76 | 90 |
| Example 8 | Polypropylene | 5.7 | 14.2 | 40 | Fresh human blood | 76 | 89 |
| Comparative Example 4 | Polybutylene terephthalate | 1.7 | 5.9 | 84 | Fresh human blood | 58 | 78 |
| Comparative Example 5 | Polypropylene | 2.1 | 6.0 | 30 | Fresh human blood | 58 | - |

### (Example 9)

The same separation material as in Example 3 was used and the same procedures were carried out, except that fresh swine bone marrow (10 mL) anticoagulated with heparin (final concentration: 50 units/mL) and CPD (final concentration: 12%) was used instead of the CPD-anticoagulated fresh bovine blood (20 mL). Table 3 and Figs. 3 and 6 show the results.

### (Example 10)

The same procedures as in Example 9 were carried out, except that a laminate of 24 layers of a polybutylene terephthalate nonwoven fabric (average fiber diameter: 5.3 µm, air permeability coefficient M: 20.0) was packed. Table 3 and Figs. 3 and 6 show the results.

### (Comparative Example 6)

The same procedures as in Example 9 were carried out, except that the separation material of Comparative Example 2 was used instead. When the syringe was pressed in the recovery process, strong resistance was felt and smooth pressing was not possible, which suggests that the inner pressure of the column was high and clogging was caused. Table 3 and Figs. 3 and 6 show the results.

**[Table 3]**

| | Nonwoven fabric material | Average fiber diameter [µm] | Air permeability coefficient M | Number of laminated layers | Biological fluid | White blood cell recovery yield [%] |
|---|---|---|---|---|---|---|
| Example 9 | Polybutylene terephthalate | 2.9 | 10.0 | 28 | Fresh swine bone marrow | 66 |
| Example 10 | Polybutylene terephthalate | 5.3 | 20.0 | 24 | Fresh swine bone marrow | 56 |
| Comparative Example 6 | Polypropylene | 2.1 | 6.0 | 30 | Fresh swine bone marrow | 32 |

### (Example 11)

The separation material of Example 3 and 12% CPD-containing fresh bovine blood (25 mL) were used. The recovery process was carried out using 10% ACD-A and 10% FBS-containing αMEM (30 mL) (viscosity: 2.9 mPa·s). Table 4 shows the results.

### (Example 12)

The experiment was carried out using the same separation material in the same manner as in Example 11, except that a 10% ACD-A and 4% human serum albumin-containing low-molecular-weight dextran injection (available from Otsuka Pharmaceutical Co., Ltd.) (viscosity: 7.3 mPa·s) was used instead. Table 4 shows the results.

### (Example 13)

The experiment was carried out using the same separation material in the same manner as in Example 11, except that a 10% ACD-A-containing SALINHES fluid solution 6% (available from Fresenius Kabi Japan) (viscosity: 2.3 mPa·s) was used instead. Table 4 shows the results.

### (Example 14)

The experiment was carried out using the same separation material in the same manner as in Example 11, except that a 10% ACD-A and 4% human serum albumin-containing SALINHES fluid solution 6% (available from Fresenius Kabi Japan) (viscosity: 4.3 mPa·s) was used. Table 4 shows the results.

### (Example 15)

The experiment was carried out using the same separation material in the same manner as in Example 11, except that 10% ACD-A-containing 20% sucrose was used instead. Table 4 shows the results.

### (Example 16)

The experiment was carried out using the same separation material in the same manner as in Example 11, except that 10% ACD-A-containing physiological saline (available from Otsuka Pharmaceutical Co., Ltd.) (viscosity: 1.1 mPa·s) was used instead. Table 4 shows the results.

**[Table 4]**

| | Detachment solution | Viscosity of detachment solution [mPa·s] | White blood cell recovery yield [%] |
|---|---|---|---|
| Example 11 | FBS-containing α MEM | 2.9 | 85 |
| Example 12 | Human serum albumin-containing low-molecular-weight dextran injection | 7.3 | 96 |
| Example 13 | SALINHES fluid solution | 2.3 | 89 |
| Example 14 | Human serum albumin-containing SALINHES fluid solution | 4.3 | 98 |
| Example 15 | Sucrose solution | - | 93 |
| Example 16 | Physiological saline | 1.1 | 84 |

The results of Examples 11 to 16 revealed that whatever detachment solution is used, the separation materials of the present invention are able to recover white blood cells at a high recovery yield.

### (Example 17)

The cells recovered in Example 8 were prepared to give a white blood cell concentration of 2×10⁶, and a 0.3-mL aliquot thereof was added to a methyl cellulose medium, MethoCult H4034 (available from StemCell Technologies) (3 mL). Then, a 1.1-mL aliquot of the mixture was dispensed onto a petri dish, and the dish was incubated at 37°C in 5% CO₂. Microscopic observation of the dish after 14 days confirmed that colonies of various hematopoietic stem cells (e. g. red blood cell progenitor cells, white blood cell progenitor cells) were formed, and the recovered cells included CD34+ cells and other hematopoietic stem cells. Fig. 8 is a photograph of the colonies of hematopoietic stem cells.

### (Example 18)

3-mL aliquots of the cell solutions recovered in Examples 9 and 10 were each combined with a 10% FBS-containing αMEM to give a total amount of 10 mL. The resulting mixtures were each inoculated on a 10 cm-diameter petri dish and then incubated at 37°C in 5% CO₂. The incubation was continued for 9 days with media exchange every 3 days, and it was then confirmed that colonies of mesenchymal stem cells were adhered to the petri dishes, and the recovered cells included mesenchymal stem cells.

### (Example 19)

A laminate of 112 layers of a polybutylene terephthalate nonwoven fabric (average fiber diameter: 3.5 µm, air permeability coefficient M: 8.9) was packed into a polycarbonate column container (thickness: 12 mm, diameter: 44 mm), as shown in Fig. 7. As shown in Fig. 10, the cell separation container was set such that the inlet was positioned below the outlet. First, physiological saline (about 50 mL) was passed through the container from the inlet to the outlet for a biological fluid. Next, CPD-anticoagulated human peripheral blood (80 mL) was passed through the container in the same direction to capture white blood cells in the cell separation container. Finally, the line was switched, and a 4% human serum albumin-containing SALINHES fluid solution 6% (available from Fresenius Kabi Japan) (viscosity: 4.3 mPa·s) was fed by pressing a syringe by hand, so that it flowed in a direction opposite to the above flow direction, in other words, from the outlet to the inlet for a biological fluid. In this manner, cells were recovered in a cell recovery bag. Table 5 shows the results.

### (Example 20)

The same procedures as in Example 19 were carried out, except that physiological saline (viscosity: 1.1 mPa·s) was used instead of the 4% human serum albumin-containing SALINHES fluid solution 6% (available from Fresenius Kabi Japan) (viscosity: 4.3 mPa·s). Table 5 shows the results.

### (Example 21)

The same procedures as in Example 19 were carried out, except that CPD-anticoagulated human umbilical cord blood was used instead of the CPD-anticoagulated human peripheral blood. Table 5 shows the results.

### (Example 22)

A laminate of 112 layers of a polybutylene terephthalate nonwoven fabric (average fiber diameter: 3.5 µm, air permeability coefficient M: 8.9) was packed into a polycarbonate column container (thickness: 12 mm, diameter: 44 mm), as shown in Fig. 7. As shown in Fig. 9, the cell separation container was set such that the inlet was positioned above the outlet. First, physiological saline (about 50 mL) was passed through the container from the inlet to the outlet for a biological fluid. Next, CPD-anticoagulated human umbilical cord blood (80 mL) was passed through the container in the same direction to capture white blood cells in the cell separation container. Finally, the line was switched, and a 4% human serum albumin-containing SALINHES fluid solution 6% (available from Fresenius Kabi Japan) (viscosity: 4.3 mPa·s) was fed by pressing a syringe by hand, so that it flowed in a direction opposite to the above flow direction, in other words, from the outlet to the inlet for a biological fluid. In this manner, cells were recovered in a cell recovery bag. Table 5 shows the results.

### (Example 23)

The same procedures as in Example 19 were carried out, except that CPD-anticoagulated bovine peripheral blood (150 mL) was used instead of the CPD-anticoagulated human peripheral blood (80 mL). Table 5 shows the results.

### (Example 24)

The same procedures as in Example 22 were carried out, except that CPD-anticoagulated bovine peripheral blood (150 mL) was used instead of the CPD-anticoagulated human umbilical cord blood (80 mL). Table 5 shows the results.

**[Table 5]**

| | Biological fluid | Detachment solution | Viscosity of detachment solution [mPa·s] | Position of inlet | White blood cell White recovery yield [%] | Mononuclear cell recovery yield [%] |
|---|---|---|---|---|---|---|
| Example 19 | Human peripheral blood | Human serum albumin-containing SALINHES fluid solution | 4.3 | Below the outlet | 78 | 83 |
| Example 20 | Human peripheral blood | Physiological saline | 1.1 | Below the outlet | 73 | 75 |
| Example 21 | Human umbilical cord blood | Human serum albumin-containing SALINHES fluid solution | 4.3 | Below the outlet | 82 | 80 |
| Example 22 | Human umbilical cord blood | Human serum albumin-containing SALINHES fluid solution | 4.3 | Above the outlet | 75 | 81 |
| Example 23 | Bovine peripheral blood | Human serum albumin-containing SALINHES fluid solution | 4.3 | Below the outlet | 93 | - |
| Example 24 | Bovine peripheral blood | Human serum albumin-containing SALINHES fluid solution | 4.3 | Above the outlet | 83 | - |

The results revealed that the separation material and the separation methods of the present invention are less likely to cause a pressure elevation, and also enable white blood cells or mononuclear cells to be efficiently separated regardless of the type of the detachment solution. The white blood cell recovery yield can be further improved by setting the cell separation container such that the inlet is positioned below the outlet. In contrast, as shown in the Comparative Examples, the use of a nonwoven fabric having a small fiber diameter and/or a small air permeability coefficient M tends to cause a pressure elevation and generally result in a decrease in the white blood cell recovery yield.

### (Example 25)

The cells separated in Examples 21 and 22 were cryopreserved and evaluated for activity after the cryopreservation. Specifically, the separated cells were transferred to a Cryobag (available from Macopharma), cooled to 4°C, and combined with a cryoprotective agent, a mixture of DMSO and dextran 40 prepared to have a final DMSO concentration of 10%. Thereafter, the temperature was controlled by a program freezer to gradually decrease in steps, and the cells were stored in a frozen state in a liquid nitrogen tank (-196°C). After 14 days, the cryopreserved cells were thawed in a warm bath at 37°C, and then transferred into a mixture of dextran and albumin. The resulting mixture was centrifuged, and the supernatant was removed. The obtained cells were resuspended in a mixture of dextran and albumin, and counted. The recovery yield after separation was calculated as the ratio of the number of cells in the treated solution after the separation to the number of cells before the separation. Additionally, the recovery yield after cryopreservation was calculated as the ratio of the number of cells in the treated solution after cryopreservation to the number of cells before cryopreservation.

The cells obtained in Example 21 were analyzed for recovery yields after separation, recovery yields after cryopreservation, and viability after cryopreservation of: white blood cells; mononuclear cells; CD34+ cells; CD133+ cells; CD34- and CD133+ cells; CD34+ and CD133+ cells; CD45+, CD133+, and CD117+ cells; CD45+, CD133-, and CD164+ cells; and CD45+ and CD117+ cells. Table 6 shows the results.

**[Table 6]**

| | Recovery yield after separation | Recovery yield after cryopreservation | Viability after cryopreservation |
|---|---|---|---|
| White blood cells | 82 | 70 | 80 |
| Mononuclear cells | 80 | 96 | |
| CD34+ cells | 86 | 80 | |
| CD133+ cells | 98 | 94 | |
| CD34- and CD133+ cells | 67 | 100 | |
| CD34+ and CD133+ cells | 100 | 87 | |
| CD45+, CD133+, and CD117+ cells | 96 | 89 | |
| CD45+, CD133-, and CD164+ cells | 89 | 100 | |
| CD45+ and CD117+ cells | 61 | 100 | |

The cells obtained in Example 22 were analyzed for recovery yields after separation, recovery yields after cryopreservation, and viability after cryopreservation of: white blood cells; mononuclear cells; CD34+ cells; CD133+ cells; CD34- and CD133+ cells; CD34+ and CD133+ cells; CD34+ and CD133- cells; CD45-, CD44+, CD73+, and CD90+ cells; CD45-, CD235a-, CD33-, and CD7- cells; CD45+, CD133+, and CD117+ cells; CD45+, CD133-, and CD117+ cells; CD45+, CD133+, and CD164+ cells; CD45+, CD133-, and CD164+ cells; CD45- and CD309+ cells; CD45+ and CD164+ cells; and CD45+ and CD117+ cells. Table 7 shows the results.

**[Table 7]**

| | Recovery yield after separation | Recovery yield after cryopreservation | Viability after cryopreservation |
|---|---|---|---|
| White blood cells | 75 | 75 | 86 |
| Mononuclear cells | 81 | 93 | |
| CD34+ cells | 100 | 100 | |
| CD133+ cells | 100 | 100 | |
| CD34- and CD133+ cells | 100 | 100 | |
| CD34+ and CD133+ cells | 100 | 99 | |
| CD34+ and CD133- cells | 100 | 100 | |
| CD45-. CD44+, CD73+. and CD90+ cells | 63 | 100 | |
| CD45-. CD235a-, CD33-, and CD7- cells | 100 | 100 | |
| CD45+. CD133+, and CD117+ cells | 100 | 79 | |
| CD45+. CD133-, and CD117+ cells | 77 | 75 | |
| CD45+. CD133+, and CD164+ cells | 100 | 100 | |
| CD45+. CD133-, and CD164+ cells | 100 | 100 | |
| CD45- and CD309+ cells | 88 | 88 | |
| CD45+ and CD164+ cells | 100 | 100 | |
| CD45+ and CD117+ cells | 94 | 95 | |

As shown above, the cell separation methods of the present invention apply very little stress to cells, and the cells obtained by the cell separation methods can maintain high activity after cryopreservation.

### REFERENCE SIGNS LIST

- 1: Inlet for biological fluid
- 2: Outlet for biological fluid
- 3: Separation material
- 4, 5: Washer for compressing separation material
- 6: Container
- 7: Blood cell component separation column
- 8: Chamber
- 9: Container packed with blood cell component separation material
- 10: Biological fluid bag
- 11: Priming solution bag (also serving as washing solution bag)
- 12: Red blood cell recovery bag
- 13: White blood cell recovery bag (mononuclear cell recovery bag)
- 14: Recovery port
- 15, 16, 17: Three-way stopcock
- 18 to 24: Line

## Claims

1. A separation material for separating white blood cells or mononuclear cells from a biological fluid, the separation material comprising a nonwoven fabric having an average fiber diameter of at least 2.0 µm but not more than 6.0 µm, and an air permeability coefficient M of at least 6.2 but not more than 35.

2. The separation material according to claim 1,
wherein the nonwoven fabric is made of at least one selected from the group consisting of polyolefins, polyamides and polyesters.

3. The separation material according to claim 1 or 2,
wherein the biological fluid is at least one selected from the group consisting of peripheral blood, umbilical cord blood, bone marrow, menstrual blood, and tissue extracts.

4. A cell separation container, comprising
a container provided with an inlet and an outlet for a biological fluid, wherein the separation material according to any one of claims 1 to 3 is packed in the container.

5. The cell separation container according to claim 4,
wherein the separation material is packed in the form of a single layer or a laminate of layers oriented in a direction of flow of the biological fluid.

6. The cell separation container according to claim 4 or 5,
wherein the separation material is packed in a state of being compressed in a direction of flow of the biological fluid.

7. The cell separation container according to any one of claims 4 to 6,
wherein the cell separation container is in the form of a column.

8. A cell separation method, comprising a step of contacting a biological fluid with the separation material according to any one of claims 1 to 3 to separate white blood cells or mononuclear cells.

9. A cell separation method, comprising:
a first step of contacting a biological fluid with the cell separation container according to any one of claims 4 to 7 to capture white blood cells or mononuclear cells on the separation material; and
a second step of recovering the captured white blood cells or mononuclear cells from the separation material using a detachment solution.

10. The cell separation method according to claim 9,
wherein the second step comprises: introducing the detachment solution through the outlet of the cell separation container, and
recovering the white blood cells or mononuclear cells through the inlet.

11. The cell separation method according to claim 9 or 10, further comprising, after the first step and before the second step, a step of introducing physiological saline or a buffer through the inlet to remove contaminant components in the cell separation container.

12. The cell separation method according to any one of claims 9 to 11, further comprising, before the first step, a step of contacting physiological saline or a buffer with the separation material.

13. The cell separation method according to any one of claims 9 to 12, further comprising, before the first step, a step of fixing the inlet for a biological fluid of the cell separation container below the outlet for a biological fluid of the cell separation container.

14. The cell separation method according to any one of claims 8 to 13,
wherein the separation material substantially captures white blood cells and platelets, and substantially does not capture red blood cells.

15. The cell separation method according to any one of claims 8 to 14,
wherein the separated white blood cells or mononuclear cells comprise hematopoietic stem cells and/or mesenchymal stem cells.

16. A method for cryopreserving cells, comprising the cell separation method according to any one of claims 8 to 15, and a step of placing the cells in a liquid nitrogen environment.

17. The cryopreservation method according to claim 16,
wherein the liquid nitrogen environment is at -196°C to -30°C.

18. The cryopreservation method according to claim 16 or 17,
wherein at least one cryoprotective agent selected from the group consisting of dimethyl sulfoxide, dextran, albumin, and hydroxyethyl starch is used.

19. The cryopreservation method according to any one of claims 16 to 18,
wherein 80% or more of cryopreserved stem cells are viable.

20. White blood cells, mononuclear cells or stem cells, obtained by the cell separation method according to any one of claims 8 to 15.

21. The stem cells according to claim 20,
which comprise cells selected from the group consisting of: CD34+ cells; CD133+ cells; CD34- and CD133+ cells; CD34+ and CD133+ cells; CD34+ and CD133- cells; CD45-, CD44+, CD73+, and CD90+ cells; CD45-, CD235a-, CD33-, and CD7- cells; CD45+, CD133+, and CD117+ cells; CD45+, CD133-, and CD117+ cells; CD45+, CD133+, and CD164+ cells; CD45+, CD133-, and CD164+ cells; and CD45- and CD309+ cells.

22. The white blood cells according to claim 20,
which comprise: CD45+ and CD164+ cells; or CD45+ and CD117+ cells.
